(19) Europäisches Patentamt
European Patent Office
Office européen des brevets

(11) Veröffentlichungsnummer : **0 167 103**
**B1**

(12) **EUROPÄISCHE PATENTSCHRIFT**

(45) Veröffentlichungstag der Patentschrift : 07.03.90

(51) Int. Cl.$^5$ : C 07 B 59/00// C07C53/21, C07C69/63, C07H5/02

(21) Anmeldenummer : 85107907.9

(22) Anmeldetag : 26.06.85

# (54) Verfahren zur Herstellung von 18F-markierten organischen Verbindungen durch nukleophilen Austausch.

(30) Priorität : 04.07.84 DE 3424525

(43) Veröffentlichungstag der Anmeldung : 08.01.86 Patentblatt 86/02

(45) Bekanntmachung des Hinweises auf die Patenterteilung : 07.03.90 Patentblatt 90/10

(84) Benannte Vertragsstaaten : BE CH DE FR GB LI NL

(56) Entgegenhaltungen :
FR–A– 2 209 584
GB–A– 2 024 222
Römpps Chemie-Lexicon (1975) 3203
T.R. DeGrado et al. J. Nucl. Med. 25, P125 (1984
E.J. Knust et al. J. Labelled Comp. and Radiopharmaceuticals 17, 353 (1980
S.J. Gatley. Int. J. Appl. Radiat. Isot. 33, 255 (1982)
S.W. Landvatter et al. Life Sciences 33, 1933 (1983)
M.S. Berridge et al. Int. J. Appl. Radiat. Isot. 34, 727 (1983)
L.A. Spitznagle et al. Frontiers in Nuclear Medicine (W. Horst et al. edit.) Springer 1980
T. Irie et al. Int. J. Appl. Radiat. Isot. 33, 1449 (1982) und 35, 517 (1984)

(73) Patentinhaber : Forschungszentrum Jülich GmbH
Postfach 1913
D-5170 Jülich (DE)

(72) Erfinder : Coenen, Heinrich-Hubert, Dr.
Mauristrasse 14a
D-4048 Grevenbroich (DE)
Erfinder : Hamacher, Kurt, Dr.
Breiniger Strasse 8
D-5100 Aachen/Kornelienmünster (DE)
Erfinder : Schüller, Manfred
Artilleriestrasse 72
D-5170 Jülich (DE)
Erfinder : Stöcklin, Gerhard, Prof. Dr.
Rödinger Strasse 18
D-5177 Titz-Kalrath (DE)
Erfinder : Klatte, Bernd, Dr.
Hagenau 65
D-2000 Hamburg 76 (DE)
Erfinder : Knöchel, Arndt, Prof. Dr.
Frahmredder 111 b
D-2000 Hamburg 76 (DE)

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

M.R. Kilbourn et al. J. Labelled Comp. and Radiopharmaceuticals 23, 1175 (1986)
T. Haradahira et al. J. Labelled Comp. and Radiopharmaceuticals 25, 498 (1988)
T.J. Tewson et al. J. Nucl. Med. 21, 559 (1980)
S.J. Gatley et al. Int. J. Appl. Radiat. Isot. 32, 211 (1981)
L.G. Hutchins et al. Int. J. Appl. Radiat. Isot. 36, 375 (1985)
N. Satyamurthy et al. Nucl. Med. Biol. 13, 617 (1986)

## Beschreibung

Die Erfindung bezieht sich auf ein Verfahren zur Herstellung $^{18}F$-markierter organischer Verbindungen der Formel

$$^{18}F—R$$

in der R ein beliebig substituierter aliphatischer, iso- oder heterocyclischer aliphatischer oder aromatischer Rest ist, wobei H-acide Verbindungen ausgenommen sind, durch Umsetzung von Verbindungen der Formel

$$X—R$$

in der X für eine nukleofuge Abgangsgruppe steht und insbesondere -Halogen, -Pseudohalogen, im Falle R = aliphatisch -OTf (Triflat) -OTos (Tosylat) und im Falle R = aromatisch $—NO_2$, $—NR_3^+$ bedeutet, mit praktisch trägerfreien Fluoridionen in Lösung.

Die Herstellung Fluor-18 markierter Verbindungen steht seit Jahren im Mittelpunkt radiopharmazeutischer Forschung für die nuklearmedizinische Funktionsdiagnostik. Dies auf Grund der optimalen nuklearen Eigenschaften von Fluor-18 für die Positron-Emissions-Tomographie. Der Positronenstrahler Fluor-18 ist auf Grund seiner Zerfallsenergie (0,64 MeV), die die höchste Auflösung bei PET-Messungen ohne störende Nebenlinien erlaubt, und auf Grund seiner Halbwertszeit von $T_{1/2}$ = 109,7 min vorteilhafter im Vergleich zu anderen Radionukliden, mit denen sich kovalent gebundene organische Verbindungen markieren lassen.

Trägerfreie bzw. markierte Verbindungen extrem hoher spezifischer Aktivität von > 1 000 Ci/mmol sind insbesondere für in-vivo Rezeptor-Studien notwendig, sowie in allen Fällen, in denen aus toxikologischen Gründen bzw. zur Erhaltung empfindlicher biologischer Gleichgewichte, eine in-vivo Applikation im subnanomolaren bzw. pikomolaren Bereich erforderlich ist. Elektrophile Verfahren basieren z. Zt. letztlich alle auf markiertem molekularen Fluor ($F_2$), das inherent inaktiven Fluorträger enthält. Die nukleophile Substitution mit Fluorid erlaubt grundsätzlich die Einführung in trägerfreier (bzw. -armer) Weise. Die Schwierigkeiten der Reaktionen mit Fluorid liegen in seiner extrem hohen Ladungsdichte und Hydrophilie. Daraus resultieren eine schwache Nukleophilie sowie hohe Adsorptionsverluste insbesondere bei Arbeiten ohne Trägerzusatz. Die Reaktionen mit trägerfreiem Fluorid setzen daher generell sorgfältigen Wasserausschluß voraus.

In bisherigen Untersuchungen wurde versucht, die Reaktivität von Ammonium- oder Alkalifluoriden durch Zugabe von Phasentransferkatalysatoren, wie langkettigen Oniumsalzen oder Kronenether, (18 Krone 6®, Firma Merck, Darmstadt) oder durch Zugabe von Silberoxid, sowie durch Verwendung leicht substituierbarer Gruppen wie Tosylaten und Triflaten zu erhöhen. Von einzelnen Fällen abgesehen, gelang die Herstellung ohne Trägerzusatz im wesentlichen nur bei einfachen Alkylverbindungen und führte auch dann nur zu kleinen Ausbeuten (< 20 %). Höhere Ausbeuten wurden nur bei Fluorethanol, Methylfluorid und einfachen aromatischen Molekülen (substituierten Benzolen), bei letzteren durch Ersatz der Nitrogruppe in DMSO, bei relativ hohen Temperaturen von 150 °C mit $Rb^{18}F$ erhalten.

Ziel der Erfindung ist daher eine Verfahrensweise, die zu höheren Ausbeuten an trägerfreien bzw. ungeträgerten $^{18}F$-Verbindungen führt. Dieses Ziel wird gemäß der Erfindung primär dadurch erreicht daß man die Substitutionsreaktion in einer Apparatur durchführt, bei der die mit der Reaktionsmischung in Berührung kommenden Flächen aus Metall oder vorzugsweise aus Glaskohlenstoff bestehen.

Als Metalle kommen insbesondere Kupfer, Edelstahl, Platin oder Inconel® in Betracht. Glaskohlenstoff ist z. B. als Sigradur®G, L oder K erhältlich.

Es hat sich überraschenderweise gezeigt, daß die Produktausbeute bei Austausch der üblichen inerten Gefäße aus Hart- oder Weichglas, Polyäthylen, Polypropylen oder Polytetrafluoräthylen durch besagte Metallgefäße erhöht ist. Eine noch wesentlich höhere Ausbeute, die fast quantitativ sein kann, wird jedoch mit Glaskohlenstoffgefäßen erreicht.

Dabei wird die Reaktion vorzugsweise entweder in einer basisches Alkalisalz, insbesondere $Na_2CO_3$, $K_2CO_3$, $Rb_2CO_3$ oder $Cs_2CO_3$, enthaltenden Acetamidschmelze bei 100 bis 150 °C durchgeführt oder insb. bei 50 bis 150 °C in homogener Phase mit einem mäßig polaren aprotischen Lösungsmittel in Gegenwart eines Komplexes von basischem Alkalisalz mit einem makrocyclischen Polyether, speziell mit Aminopolyether-(Kryptofix®2.2.2)-komplexiertem $K_2CO_3$ — insbesondere in Acetonitril und/oder DMSO als Lösungsmittel.

Gemäß der Erfindung werden Nebenreaktionen unterdrückt, Wandverluste nahezu vollständig vermieden und überraschend hohe Ausbeuten (bis 95 %) erreicht. Die Reaktion in aprotischen Lösungsmitteln mit Aminopolyether ist darüber hinaus durch Spuren von Wasser (bis ca. 1 %) nur unwesentlich beeinträchtigt.

Die Erfindung umfaßt folgende Verbesserungen :

1) Ausschaltung von Adsorptionsverlusten

Da stets hohe Adsorptionsverluste bei der Reaktion von Fluorid ohne Trägerzusatz auftreten, wird die Gesamtausbeute (bezogen auf das im Kernreaktor oder Beschleuniger produzierte $^{18}F$-Fluorid) üblicherweise drastisch verringert. Systematische Studien verschiedener Wandmaterialien für Reaktionsgefäße ergaben nun, daß Kupfer, Edelstahl und Inconel® (ca. 10 % Wandverluste) und insbesondere Glaskohlenstoff (< 2 % Wandverlust) eine ungeträgerte $^{18}F$-Fluorierung obiger Verbindungen mit radiochemischen Gesamtausbeuten von 60-95 % unter milderen Reaktionsbedingungen als bisher bekannt ermöglichen. Fluorierungen in den üblicherweise verwendeten Teflongefäßen führen dagegen zwar ebenfalls zu geringen Wandverlusten jedoch zu kleineren Produktausbeuten. Die Metallgefäße und solche aus Glaskohlenstoff bieten zudem in Anbetracht der guten Wärmeleitfähigkeit bessere Möglichkeiten der Temperatureinstellung und kürzere Synthesezeiten und damit geringere Verluste von Radioaktivität.

2) Verbesserung der Umsetzung in Gegenwart von Alkalisalz in einer Acetamidschmelze oder insbesondere in homogenen Aminopolyetherlösungen

A. Durch Zusatz von basischen Alkalisalzen geringer nukleophilie, insbesondere $Na_2CO_3$, $K_2CO_3$, $Rb_2CO_3$, $Cs_2CO_3$ zu einer Acetamidschmelze bei 100-150 °C konnte der Austausch des gelösten Fluorids ohne Trägerzugabe mit Alkylverbindungen von 0 % auf 70 % radiochemische Ausbeute gesteigert werden.

B. Alternativ wurden in homogener Lösung in insbesondere Acetonitril, das einen Komplex des [2.2.2] Cryptanden (Kryptofix®) mit $K_2CO_3$ enthielt, ebenfalls 60 % bis 95 % von ungeträgertem $^{18}F$-Fluorid mit den gleichen Alkylverbindungen am Rückfluß umgesetzt. Die besondere Eignung dieser Reaktionsmischung liegt in der optimalen Kation-Polyether Kombination (Komplexe aus anderen Metallkationen und entsprechenden Polyethern waren ähnlich gut geeignet) sowie in der Verwendung äquimolarer Mengen des Aminopolyethers und der Alkalikationen. Dies konnte insbesondere mit langkettigen ω-Halogenfettsäuren und geeignet geschützten Zuckern (von beiden Verbindungsklassen sind bedeutende $^{18}F$-markierte Radiopharmaka für PET bekannt; siehe Ausführungsbeispiele) gezeigt werden.

Diese Reaktionslösungen (A und B) sind ebenso für den nukleophilen Fluoraustausch mit aromatischen Verbindungen geeignet, wobei gegenüber bislang bekannten Methoden auch Halogene als Abgansgruppen (neben $-NO_2$) und die niedrigere Reaktionstemperatur wesentliche Vorteile darstellen. Dies ist besonders wichtig zur trägerfreien Herstellung von komplexen $^{18}F$-Rezeptorliganden zur in-vivo Erfassung von Rezeptordichten mittels PET. Ein wichtiges Beispiel stellen Neuroleptika der Butyrophenonreihe dar wie Spiroperidol, Methylspiroperidol, Benperidol usw., die ein Fluoratom in der pharmakophoren Gruppe tragen. Die zu erhaltenden spezifischen Aktivitäten für alle beschriebenen Verbindungen sind $> 1{,}5 \times 10^4$ Ci/mmol.

## Beispiel 1

[$^{18}F$]-17-Fluorheptadekansäure (17-$^{18}F$-Hds) zu A: Acetamidschmelze

2-5 ml $^{18}F$-enthaltendes Wasser (bidest) und 10 mg $K_2CO_3$ wurden bei 180 °C im Helium-strom (ca. 50 ml/min) i-n einem zylindrischen Sigradur®-G-Gefäß von ca. 10 ml Fassungsvermögen zur Trockne eingedampft, anschließend noch 15 min bei 180 °C im Heliumstrom ausgeheizt und im He-Strom abgekühlt. Nach Zugabe von 100 mg Acetamid und 20-120 mg 17-Brom-Heptadekansäuremethylester wurde das Reaktionsgefäß mittels V4A-Stopfen und Sigraflex®-Dichtung verschlossen. Die Reaktionszeit betrug 10-20 min bei 100-150 °C unter Rühren im Ölbad. Nach Abkühlen wurden 2 ml 5n methanolische KOH hinzugefügt und die Mischung 15 min lang auf 70 °C unter Rühren im Ölbad erwärmt.

Nach Zugabe von 10 ml $H_2O$ und 3 ml 15 %-iger $H_2SO_4$ folgte mehrmaliges Extrahieren mit 10 bzw. 5 ml n-Heptan bei 80 °C. Die Heptanphasen wurden zur Trockne eingedampft und die [$^{18}F$]-17-Fluoroheptadekansäure in 2 ml Eluens aufgenommen und mittels Hochdruckflüssigkeitschromatographie isoliert.

HPLC: Nucleosil C-18; 7,5 μm; 25 cm; 16 mm i. Ø; Methanol: Wasser: Essigsäure 896 : 100 : 4 (v, v, v); 43 bar; 7,4 ml/min; k' (17-F-Hds) = 2,64 Ausbeute an 17-$^{18}F$-Hds: 48 ± 4 %

## Beispiel 2

zu B: Aminopolyether-Acetonitrillösung

2 bis 5 ml $^{18}F$-Ionen enthaltendes Wasser (bidest) und 2,3 mg $K_2CO_3$ (p.a.) sowie 12,6 mg [2.2.2] Cryptand wurden in ein zylindrisches Sigradur®-G-Gefäß von ca. 10 ml Fassungsvermögen gegeben.

Bei einer Ölbadtemperatur von ca. 110 °C und einer Temperatur von ca. 90 °C im Reaktionsgefäß wurde die Lösung in einem Heliumstrom von ca. 50 ml/min zur Trockne eingedampft. Anschließend wurden 0,5 ml $CH_3CN$, Uvasol (getrocknet über $Al_2O_3$, Aktivitätsstufe I, aktiv, neutral) und 10 mg 17-Brom-Heptadekansäuremethylester zugegeben un die Mischung 10 min am Rückfluß gekocht. Danach erfolgte eine Zugabe von 2 ml 5 n methanolischer KOH und 15 min Kochen am Rückfluß. Die weitere Aufarbeitung erfolgte wie bei der Acetamidschmelze beschrieben. Ausbeute: 92 ± 3 %

Beispiel 3

[$^{18}$F]-2-Fluor-2-deoxy-D-glucose (2-$^{18}$FDG) zu B : Aminopolyether-Acetonitrillösung.

Das $^{18}$F-enthaltende Wasser wurde wie in Beispiel 2 beschrieben in Gegenwart von [2.2.2] Cryptand und $K_2CO_3$ zur Trocke eingedampft. Anschließend wurde eine Lösung von 20 mg 1,3,4,6-Tetra-O-acetyl-2-O-trifluormethansulfonyl-β-D-mannopyranose (getrocknet über Sicapent) in 1 ml Acetonitril (siehe Beispiel 2) zugegeben und die Mischung 5 min zum Sieden erhitzt. Danach wurde die Lösung auf ungefähr 0,5 ml im He-Strom eingedampft und nach Verdünnung mit ca. 5 ml Wasser durch eine SEP-PAK $C_{18}$ Patrone permeiert. Das Produkt/Edukt Gemisch wurde mit 2 ml THF aus der Patrone extrahiert und die THF-Phase zur Trockne eingedampft. Der Rückstand wurde nach Zugabe von 2 ml 1 molarer Salzsäure 15 min unter Rückfluß hydrolysiert. Zur Reinigung des Produktes von hydrophoben Nebenprodukten, HCl und unreagiertem $^{18}$F- wurde die Lösung von 2-$^{18}$FDG über eine $C_{18}$SEP PAK Patrone filtriert und anschließend über eine Kurzsäule mit Ionenretardierungsharz (AG 11AB, BioRad) und Aluminiumoxid chromatographiert. Produkt-Ausbeute 82 ± 4 %.

**Patentansprüche**

1. Verfahren zur Herstellung $^{18}$F-markierter organischer Verbindungen der Formel

$$^{18}F—R$$

in der R ein beliebig substituierter aliphatischer, iso- oder heterocyclischer aliphatischer oder aromatischer Rest ist, wobei H-acide Verbindungen ausgenommen sind, durch Umsetzung von Verbindungen der Formel

$$X—R$$

in der X für eine nukleofuge Abgangsgruppe steht und insbesondere -Halogen, -Pseudohalogen, im Falle R = aliphatisch -OTf (Triflat), -OTos (Tosylat) und im Falle R = aromatisch —$NO_2$, —$NR_3^+$ bedeutet, mit praktisch trägerfreien — Fluoridionen in Lösung, dadurch gekennzeichnet, daß man die Substitutionsreaktion in einer Apparatur durchführt, bei der die mit der Reaktionsmischung in Berührung kommenden Flächen aus Metall oder vorzugsweise Glaskohlenstoff bestehen.

2. Verfahren nach Anspruch 1, dadurch gekennzeichnet, daß die Flächen aus Kupfer, Edelstahl, Platin oder Nickelbasislegierungen bestehen.

3. Verfahren nach Anspruch 1 oder 2, dadurch gekennzeichnet, daß die Reaktion in einer basisches Alkalisalz, insbesondere $Na_2CO_3$, $K_2CO_3$, $Rb_2CO_3$ oder $Cs_2CO_3$, enthaltenden Acetamidschmelze bei 100 bis 150 °C durchgeführt wird.

4. Verfahren nach Anspruch 1, dadurch gekennzeichnet, daß man die Substitutionsreaktion in homogener Lösung in einem mäßig polaren, aprotischen Lösungsmittel in Gegenwart eines darin gelösten Komplexes eines makrocyclischen Polyethers mit einem basischen, nicht nukleophilen Alkalisalz bei einer Temperatur im Bereich von 50 bis 150 °C durchführt.

5. Verfahren nach Anspruch 4, dadurch gekennzeichnet, daß als Lösungsmittel Acetonitril verwendet wird.

6. Verfahren nach Anspruch 4 oder 5, dadurch gekennzeichnet, daß der Komplex aus einem makrocyclischen Aminopolyether mit einem Alkalisalz gebildet wird.

7. Verfahren nach Anspruch 6, dadurch gekennzeichnet, daß der Komplex aus [2.2.2] Cryptand mit $K_2CO_3$ gebildet wird.

8. Vorrichtung zur $^{18}$F-Markierung organischer Verbindungen durch nukleophilen Austausch einer nukleofugen Gruppe durch $^{18}$F-Fluorid in einer Substitutionsreaktion, dadurch gekennzeichnet, daß die mit der reagierenden Mischung in Berührung kommenden Flächen aus Glaskohlenstoff bestehen.

**Claims**

1. Process for the preparation of fluorine-18 labelled organic compounds of the formula

$$^{18}F—R$$

wherein R is an optionally substituted aliphatic, isocyclic or heterocyclic aliphatic or aromatic radical, excluding H acid compounds, by reaction of compounds of the formula

$$X—R$$

wherein R stands for a nucleofugal origin group and signifies especially -halogen, -pseudohalogen, in the

event that R = aliphatic -OTf (triflate), -OTos (tosylate), and in the event that R = aromatic —$NO_2$, $NR_3^+$, with substantially carrier-free $^{18}$fluoride ions in solution, characterised in that the substitution reaction is carried out in an apparatus wherein those surfaces which come into contact with the reaction mixture are made of metal or preferably glass carbon.

2. Process according to claim 1, characterised in that the surfaces are made of copper, special steel, platinum, or nickel-based alloys.

3. Process according to claim 1 or 2, characterised in that the reaction is carried out in an acetamide melt containing a basic alkali salt especially $Na_2CO_3$, $K_2CO_3$, $Rb_2CO_3$, or $Cs_2CO_3$, at 100 to 150 °C.

4. Process according to claim 1, characterised in that the substitution reaction is carried out in a homogeneous solution in a moderately polar, aprotic solvent in the presence of a complex, in solution therein, of a macrocyclic polyether with a basic, non-nucleophilic alkali salt at a temperature in the range 50 to 150 °C.

5. Process according to claim 4, characterised in that acetonitrile is used as solvent.

6. Process according to claim 4 or 5, characterised in that the complex is formed of a macrocyclic aminopolyether with an alkali salt.

7. Process according to claim 6, characterised in that the complex is formed of [2,2,2] cryptand with $K_2CO_3$.

8. Apparatus for the fluorine-18 labelling of organic compounds by nucleophilic substitution of a nucleofugal group with $^{18}F$ fluoride in a substitution reaction, characterised in that the surfaces which come into contact with the reacting mixture are made of glass carbon.

**Revendications**

1. Procédé de préparation de composés organiques marqués au fluor-18 de formule

$$^{18}F—R$$

dans laquelle R est un radical aliphatique, iso- ou hétéro-cyclo-aliphatique ou -aromatique éventuellement substitué, en excluant les composés à hydrogène acide, par réaction de composés de formule

$$X—R$$

dans laquelle X signifie un groupe de départ nucléofuge et notamment halogène, pseudo-halogène, dans le cas où R est aliphatique, -OTf (trifluorométhylsulfonate) -OTos (tosylate) et, dans le cas où R est aromatique, —$NO_2$, —$NR_3^+$, sur des ions fluorure-$^{18}$ pratiquement sans support et en solution, caractérisé en ce qu'il consiste à effectuer la réaction de substitution dans un appareillage dans lequel les surfaces venant en contact avec le mélange réactionnel sont en métal ou, de préférence, en carbone vitreux.

2. Procédé suivant la revendication 1, caractérisé en ce que les métaux sont en cuivre, en acier fin, en platine ou en alliages à base de nickel.

3. Procédé suivant la revendication 1 ou 2, caractérisé en ce qu'il consiste à effectuer la réaction entre 100 et 150 °C dans un acétamide fondu contenant un sel de métal alcalin basique, notamment $Na_2CO_3$, $K_2CO_3$, $Rb_2CO_3$ ou $Cs_2CO_3$.

4. Procédé suivant la revendication 1, caractérisé en ce qu'il consiste à effectuer la réaction de substitution en solution homogène dans un solvant modérément polaire et aprotique, en la présence d'un complexe dissous en son sein d'un polyéther macrocyclique et d'un sel de métal alcalin basique non nucléophile, en opérant à une température de l'ordre de 50 à 150 °C.

5. Procédé suivant la revendication 4, caractérisé en ce qu'il consiste à utiliser de l'acétonitrile comme solvant.

6. Procédé suivant la revendication 4 ou 5, caractérisé en ce qu'il consiste à former le complexe d'un aminopolyéther macrocyclique et d'un sel de métal alcalin.

7. Procédé suivant la revendication 6, caractérisé en ce qu'il consiste à former le complexe à partir de [2,2,2]-cryptate et de $K_2CO_3$.

8. Appareillage de marquage au $F^{18}$ de composés organiques, par remplacement nucléophile d'un groupe nucléofuge par du fluor-$F^{18}$ dans une réaction de substitution, caractérisé en ce que les surfaces venant en contact avec le mélange réactionnel sont en carbone vitreux.